# FASCICULE DE BREVET EUROPEEN

(11) **EP 1 594 832 B1**
(45) Date de publication et mention de la délivrance du brevet: **10.01.2007**
(21) Numéro de dépôt: 04713099.2
(22) Date de dépôt: 20.02.2004
(51) Int. Cl.: C07C 45/89, C07C 49/88

(54) **PROCEDE DE PREPARATION DU DIMETHYLCETENE PUIS DU POLYDIMETHYLCETENE A PARTIR DE L'ANHYDRIDE ISOBUTYRIQUE**
VERFAHREN ZUR HERSTELLUNG VON DIMETHYLKETEN UND POLYDIMETHYLKETEN AUS ISOBUTTERANHYDRID
METHOD OF PREPARING DIMETHYLCETENE AND, SUBSEQUENTLY, POLYDIMETHYLCETENE FROM ISOBUTYRIC ANHYDRIDE

(30) Priorité: 21.02.2003 FR 0302219
(43) Date de publication de la demande: 16.11.2005
(73) Titulaire: Arkema France, 92800 Puteaux (FR)
(72) Inventeur: HUB, Serge, F-69100 Villeurbanne (FR); MARTINO-GAUCHI, Georges, F-69500 Chantilly (FR); LINEMANN, Reinhard, F-27300 Bernay (FR); LE, Guillaume, F-14000 Caen (FR)
(74) Mandataire: Mouttet, Marie-Paule
(86) Numéro de dépôt international: PCT/FR2004/000399
(87) Numéro de publication internationale: WO 2004/076508

(56) Documents cités:
- US-A- 3 201 474
- US-A- 5 169 994
- US-A- 5 258 556
- PREGAGLIA G; BINAGHI M: "Polymerisation of dimethylketene. A. Dimethylketene by pyrolysis of isobutyric anhydride" MACROMOLECULAR SYNTHESES, vol. 1968, no. 3, 1969, pages 152-160, XP0009016143

## Description

La présente invention concerne un procédé de préparation du diméthylcétène (abrégé DMK dans la suite du texte) puis du polydiméthylcétène (abrégé PDMK dans la suite du texte) par polymérisation du DMK. Plus précisément, le DMK est obtenu par pyrolyse de l'anhydride isobutyrique (abrégé ANIB dans la suite du texte) ce dernier se décomposant sous l'effet de la chaleur (pyrolyse) en acide isobutyrique (abrégé AIB dans la suite du texte) et DMK. Une mole d'ANIB pyrolisée donne une mole d'AIB et une mole de DMK.

Le PDMK est barrière aux gaz et en particulier à l'oxygène et de plus il possède des propriétés barrières à l'humidité. Il est utile pour fabriquer des structures monocouches ou des structures multicouches comprenant une couche de PDMK et au moins une couche d'un autre matériau. Ces structures sont utiles pour fabriquer en particulier des emballages alimentaires devant être stérilisés ou pasteurisés.

Le brevet US 5169994 décrit un procédé pour la fabrication de 2,2,4,4-tétraméthylcyclobutane-1,3-diol comprenant les étapes consistant :
(1) à introduire de l'ANIB dans une zone de pyrolyse, dans laquelle l'ANIB est chauffé à une température de 350 à 600°C pour produire un effluent de vapeur comprenant du DMK, de l'AIB et de l'ANIB n'ayant pas réagi;
(2) à refroidir rapidement l'effluent de vapeur pour condenser l'AIB et l'ANIB et séparer le condensat de la vapeur de DMK;
(3) à introduire la vapeur de DMK dans une zone d'absorption dans laquelle la vapeur de DMK est mise en contact avec et dissoute dans un solvant comprenant un ester contenant de 4 à 40 atomes de carbone et constitué du produit de réaction d'un acide carboxylique aliphatique et d'un alcool pour produire un effluent comprenant une solution de DMK dans le solvant;
(4) à introduire l'effluent de la zone d'absorption dans une zone de dimérisation dans laquelle l'effluent est chauffé à une température de 70 à 140°C pour convertir le DMK en 2,2,4,4- tétraméthylcyclobutane-1,3-dione pour produire un effluent comprenant une solution de 2,2,4,4-tétraméthylcyclobutane-1,3-dione dans le solvant ; et
(5) à introduire l'effluent de la zone de dimérisation dans une zone d'hydrogénation, dans laquelle l'effluent est mis en contact avec un catalyseur d'hydrogénation dans des conditions de pression et de température d'hydrogénation pour produire un effluent comprenant une solution de 2,2,4,4-tétraméthylcyclobutane-1,3-diol dans le solvant.

Dans la description, il est précisé qu'on alimente l'étape de pyrolyse avec de l'ANIB habituellement en mélange avec un gaz inerte et sous une pression réduite telle que 20 à 500 Torr. On ne sait pas si la pression réduite concerne l'ANIB à cause de la présence de gaz inerte ou si c'est le mélange d'ANIB et de gaz inerte qui est sous pression réduite. Dans l'exemple 1, la pyrolyse de l'ANIB est effectuée à 250 torr et le gaz récupéré, après séparation de l'AIB et de l'ANIB, est constitué de 97% de DMK. C'est donc qu'il n'y avait pas de gaz de dilution. Le taux de conversion de l'ANIB dans la pyrolyse, c'est à dire le rapport de la quantité d'ANIB pyrolysée sur la quantité d'ANIB entrant dans la zone de pyrolyse, est de 60%.

Le brevet **US 5258256** décrit un procédé très semblable au précédant mais les conditions de la pyrolyse de l'ANIB quant à la présence de gaz inerte ne sont pas plus claires. Il est seulement montré dans les exemples que la pression de la pyrolyse est de 87, 105 ou 123 Torr.

On a maintenant trouvé qu'en effectuant la pyrolyse en présence d'un gaz inerte à pression atmosphérique, donc dans des conditions opératoires simples, on obtenait un taux de conversion de l'ANIB d'au moins 80%, habituellement entre 80 et 95% (dépendant des conditions réactionnelles).

Le courant de sortie de la pyrolyse est constitué de DMK, de gaz inerte, d'AIB et/ou d'ANIB n'ayant pas réagi. Ce courant est refroidi pour séparer le DMK et le gaz inerte de l'AIB et/ou de l'ANIB. On obtient un courant de DMK et de gaz inerte contenant encore un peu d'AIB. Dans les procédés de l'art antérieur cités plus haut, ce courant de DMK est absorbé dans un solvant de type ester puis ce courant de solvant contenant le DMK est introduit dans une zone de dimérisation. On a maintenant trouvé que le courant de DMK contenant encore un peu d'AIB et obtenu par refroidissement du courant de sortie de la pyrolyse peut être lavé pour débarrasser le DMK de toute trace d'AIB. On peut ainsi obtenir le DMK avec une pureté telle qu'on peut le polymériser en PDMK ensuite.

La présente invention concerne un procédé de préparation de diméthylcétène (DMK) par pyrolyse de l'anhydride isobutyrique (ANIB) dans lequel :
a) on préchauffe à pression atmosphérique entre 300 et 340°C un mélange comprenant 1 à 50% en volume d'ANIB pour respectivement 99 à 50% de gaz inerte,
b) puis ce mélange est porté à une température comprise entre 400 et 550°C pendant un temps de contact compris entre 0,05 et 10 s pour obtenir un mélange de DMK, de gaz inerte, d'acide isobutyrique (AIB) voire d'ANIB n'ayant pas réagi,
c) le mélange issu de b) est refroidi (étape de condensation dudit mélange) pour séparer le mélange gazeux comprenant le DMK de l'AIB et/ou de l'ANIB condensés.
   S'agissant de la pyrolyse de l'ANIB, on peut utiliser tout dispositif permettant de mélanger l'ANIB et un gaz inerte puis de porter le mélange aux températures requises. Ces dispositifs sont connus en eux mêmes. Le gaz inerte signifie tout gaz qui n'est pas altéré pendant la pyrolyse de l'ANIB. A titre d'exemples, on peut citer l'azote et l'hélium.
   Quant aux proportions d'ANIB et de gaz inerte, elles sont avantageusement de 5 à 50% en volume d'ANIB pour respectivement 95 à 50% de gaz inerte et de préférence de 8 à 21% en volume d'ANIB pour respectivement 92 à 79% de gaz inerte.
   Quant au temps de contact, il est avantageusement compris entre 0,15 et 0,25 s. On obtient généralement un taux de conversion de l'ANIB, c'est à dire le rapport de la quantité d'ANIB pyrolysée sur la quantité d'ANIB entrant dans la zone de pyrolyse, de l'ordre de 80 à 95%. La sélectivité de la pyrolyse, c'est à dire le rapport (nombre de moles de DMK obtenu) / (nombre de moles d'ANIB pyrolysé), est de l'ordre de 100%.
   Quant à l'étape c), on peut utiliser tout dispositif permettant de refroidir des gaz et de séparer une phase gazeuse d'une phase liquide. Ces dispositifs sont connus en eux-mêmes. Il est recommandé de refroidir le système le plus vite possible pour éviter, du moins limiter, la recombinaison du DMK avec l'AIB qui redonne de l'ANIB.
   Le mélange gazeux comprenant le DMK (gaz inerte véhiculant essentiellement du DMK gazeux) et issu de l'étape c) peut encore contenir de faibles proportions d'AIB et/ou d'ANIB. Selon donc une forme avantageuse de l'invention, ce mélange gazeux est mis en contact avec une solution de lavage pour purifier le DMK en réduisant voire en éliminant toute trace d'AIB et/ou ANIB. Le lavage du mélange gazeux comprenant le DMK et issu de l'étape c) s'effectue avantageusement dans une colonne de lavage remplie de garnissages structurés, ladite colonne étant avantageusement munie d'un dévésiculeur (disponible commercialement), positionné soit en tête de colonne, soit en pied de colonne. De préférence, le dévésiculeur est situé en tête de colonne. Le dévésiculeur permet, le cas échéant, d'arrêter les vésicules (sorte de brouillard) présentes dans le courant gazeux de DMK entrant dans la colonne de lavage à une température située aux environs de 10 à 50°C et qui interféreraient sur la purification du DMK. Ce mélange gazeux entre en pied de colonne de lavage, alors que le solvant de lavage entre en tête de colonne.
   Avantageusement, on utilise une colonne à plateaux ou contenant un garnissage. Les dimensions sont déterminées facilement par l'homme de l'art en fonction des propriétés des gaz et de la solution de lavage.
   La solution de lavage peut être l'ANIB, un hydrocarbure saturé ou insaturé, aliphatique ou alicyclique et substitué ou non substitué dont la liste est donnée plus loin dans le texte dans la rubrique « solvant de polymérisation », Dans le cas de l'utilisation du solvant de polymérisation, le DMK solubilisé lors de cette étape de polymérisation est strippé de la phase liquide par un courant gazeux inerte en pied de colonne. L'homme de l'art détermine les conditions de pression et de température pour que la solution de lavage absorbe préférentiellement l'AIB et qu'on obtienne un courant de DMK et de gaz inerte contenant le moins possible d'AIB voire n'en contenant plus. Si la solution de lavage a aussi absorbé du DMK, on peut aussi la faire passer dans une colonne de strippage à basse pression pour récupérer le DMK sans stripper l'AIB. On utilise de manière avantageuse l'ANIB comme solvant de lavage.
   On obtient, à l'issu de l'étape de lavage, du DMK avec une pureté suffisante pour le polymériser. On entend de façon générale par "pureté suffisante pour le polymériser", un DMK ayant une teneur en AIB inférieure ou égale à 2000ppm, sachant que moins il y a d'AIB et moins on consommera de catalyseur pour la polymérisation par la suite.
   Le DMK gazeux (entraîné par le gaz porteur inerte) est quasiment voire complètement débarrassé de toute trace d'AIB et/ou d'ANIB, la solution de lavage ayant entraîné en grande partie l'AIB et/ou l'ANIB résiduel(s). A l'issue de cette étape de lavage, on obtient un DMK pur à plus de 98% en mol, avantageusement à plus de 99%, avec des traces d'AIB < ou = à 0,2% en mol et des traces d'ANIB < ou = à 1% en mol, avantageusement < ou = à 0,5%.
   Selon une autre forme de l'invention :
d) le mélange gazeux comprenant le DMK préparé précédemment est absorbé dans un solvant de type hydrocarbure saturé ou insaturé, aliphatique ou alicyclique et substitué ou non substitué,
e) puis dans ce solvant contenant le DMK, on effectue la polymérisation du DMK en PDMK en présence d'un système d'amorçage cationique comprenant un amorceur (I), un co-amorceur (K) et un agent complexant (CoK),
f) à la fin de la réaction, on élimine le DMK qui n'a pas réagi et on sépare le PDMK du solvant et des résidus du système d'amorçage.

La présence de deux doubles liaisons adjacentes carbone-carbone et carbone-oxygène confère au diméthylcétène une réactivité très importante. Il est intéressant d'orienter sélectivement l'ouverture de l'une ou l'autre des doubles liaisons afin de promouvoir une polymérisation régulière des unités monomère (A) conduisant à des polymères de structures β-cétonique (PolyA) ou une polymérisation régulière des unités monomère (B) conduisant à des polymères de structures de type polyacétal vinylique (PolyB), voire à l'addition alternée des unités (A) et (B) conduisant à un polyester vinylique (PolyAB).

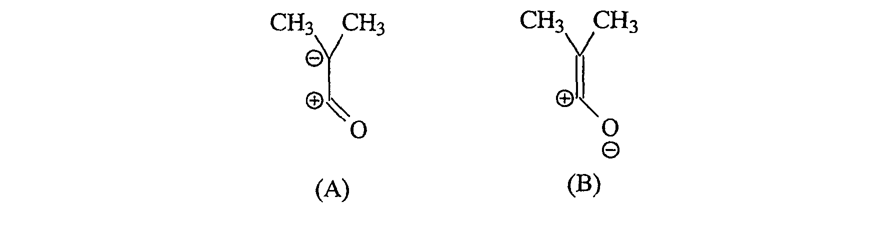

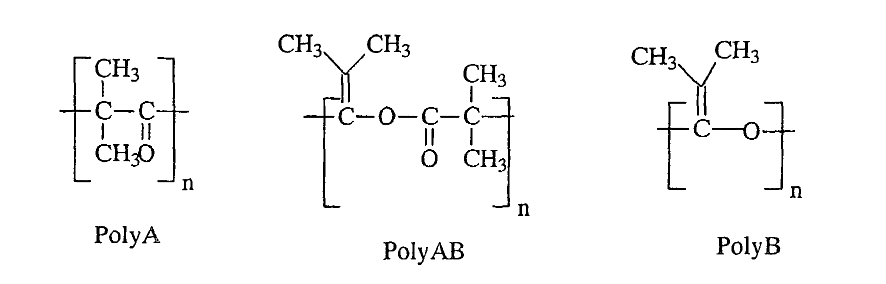

Ces conditions opératoires de la polymérisation du DMK permettent d'orienter sélectivement la polymérisation vers la formation d'un polymère de structure β-cétonique avec de très bons rendements >65% et en présence de solvants usuels, peu onéreux.

De plus, pour une meilleure efficacité et une meilleure reproductibilité, la demanderesse s'est placée en amorçage cationique homogène. Ce procédé n'entraîne pas la formation de peroxydes et permet une production sans danger du PDMK. De plus on n'observe une limitation des réactions de transfert qui entraînent classiquement la formation de chaînes de masse moléculaire faible et des rendements peu élevés. Ces différents paramètres permettent une synthèse à grande échelle de PDMK.

S'agissant du solvant de polymérisation, il est de type hydrocarbure saturé ou insaturé, aliphatique ou alicyclique et substitué ou non substitué. Les solvants utilisés sont les solvants classiques de la polymérisation cationique connus de l'homme de l'art. On peut citer à titre d'exemple les hydrocarbures tels que les hexanes, les heptanes, le toluène, le méthylcyclohexane, l'éthylcyclohexane, le propylcyclohexane, les chlorures d'alkyl (halogéné primaire et secondaire) tel que le chlorure de méthylène, le chlorure d'éthyle, le chlorure de propyle, le chlorure de butyle, le chlorure de pentyle, le chlorure d'hexyle, le chlorobenzène, le dichlorométhane, le chloroforme et les mêmes composés avec un ou des atomes (selon les cas) de brome à la place du ou des atomes de chlore, les hydrocarbures non aromatiques nitrés tel que le nitrométhane, le nitroéthane et le nitropropane. Toutefois, les solvants non toxiques et non polluants seront généralement préférés.

S'agissant de l'absorption du DMK par le solvant de polymérisation, on peut utiliser tout dispositif de mise en contact d'un gaz et d'un liquide. Avantageusement, on utilise une colonne à plateaux ou contenant un garnissage. Les dimensions sont déterminables facilement par l'homme de l'art en fonction des propriétés des gaz et du solvant. L'homme de l'art peut déterminer facilement les conditions de pression et de température pour absorber le DMK ayant une pureté suffisante pour le polymériser et pas le gaz inerte vecteur du mélange gazeux.

Le solvant dans lequel a lieu la polymérisation a un rôle important. Il doit non seulement favoriser la séparation des charges mais également solvater les chaînes en croissance pour ralentir la précipitation tout en ne gênant pas l'approche du monomère par la formation de cage à solvant. Si les solvants polaires favorisent la dissociation des paires d'ions par leur constante diélectrique élevée et donc augmentent la proportion d'ions libres réactifs, ils solvatent également préférentiellement les centres actifs et donc limitent les conversions en gênant l'approche du monomère. De manière générale, il ne faut pas qu'il y ait d'entraves à la solvatation des centres actifs par le DMK. Dans un solvant apolaire ou moyennement polaire, le DMK solvatera préférentiellement les chaînes en croissance mais les réactions de transfert seront favorisées par le solvant, l'emploi d'un agent complexant permet alors de limiter ces réactions afin d'obtenir des masses molaires élevées.

Le solvant peut, après séparation du PDMK, être réutilisé pour absorber du DMK soit en discontinu soit en tournant en boucle dans un procédé continu. Le système catalytique peut être introduit avant ou après l'absorption du DMK dans le solvant.

S'agissant du système d'amorçage cationique, il comprend un amorceur (I), un co-amorceur (K) et un agent complexant (CoK).

Selon un mode de réalisation, le système d'amorçage est caractérisé en ce que l'agent complexant (CoK) est un agent libérant le centre actif de polymérisation de son contre-anion généré par la réaction entre le co-amoceur (K) et l'amorceur (I).

Selon un mode de réalisation, l'agent complexant (CoK) est une molécule possédant au moins une double liaison appauvrie en électron par un groupement électro-attracteur.

Selon un mode de réalisation, le co-amorceur (K) est un acide de Lewis de formule générale RₙMX₃₋ₙ pour M un élément appartenant à la colonne IIIA, de formule générale MX₄ pour M un élément appartenant aux colonnes VA, IVA et IVB et de formule générale MX₅ pour M un élément appartenant à la colonne VB avec :
- R un radical monovalent pris dans le groupe contenant les groupements hydrocarbonés de 1 à 12 atomes de carbone de type alkyl, aryl, arylalkyl, alkylaryl, cycloalkyl et des alkoxy ;
- X un atome d'halogène pris dans le groupe F, Cl, Br et I ;
- n un nombre entier de 0 à 3.

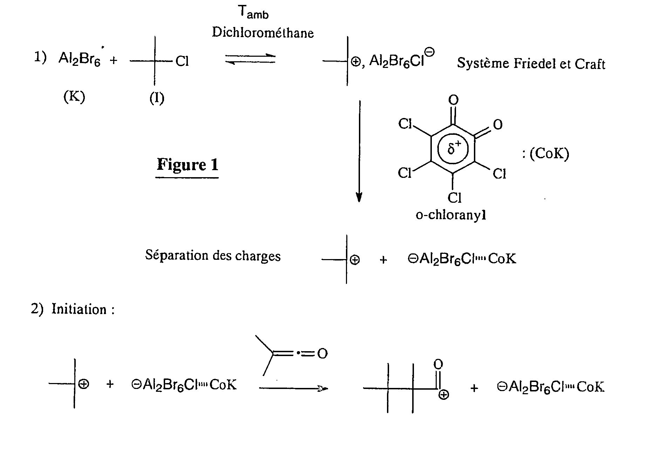

La **figure 1** représente la phase d'amorçage de la polymérisation cationique avec AlBr₃ comme co-amorceur (k), le chlorure de tertiobutyle comme amorceur (I) et l'o-chloranyl comme agent complexant (CoK).

Les avantages du système d'amorçage sont les suivants :
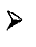 le système catalytique ainsi généré soit avant la polymérisation ou in-situ permet d'éviter la formation de trimère qui se produit lors de la polymérisation du DMK en présence d'un acide de Lewis seul. En effet, l'espèce qui amorce la polymérisation produit une extrémité neutre qui évite ainsi la formation du zwitterion intermédiaire au trimère. Ce procédé permet donc de travailler dans des solvants apolaires ou de polarités moyennes dont la toxicité est compatible avec une utilisation à grande échelle contrairement aux solvants polaires cités plus haut, sans formation de trimère.
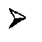 Le système d'amorçage permet de contrôler la nature des bouts de chaînes en choisissant la nature de l'amorceur. On peut ainsi introduire en bout de chaîne une fonctionnalité non réactive en polymérisation cationique mais qui permet une modification ultérieure du polymère. D'autre part, on peut aussi générer des polymères branchés ou en étoile en utilisant un amorceur de fonctionnalité supérieure à 2.
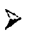 L'agent complexant (CoK) du système catalytique (I+K+CoK) permet selon sa nature de solubiliser le co-amorceur (K) même en milieu faiblement polaire et soluble même à des concentrations fortes de l'ordre de 1 M d'acide de Lewis en tant que co-amorceur (K) alors qu'il est généralement difficile de solubiliser cet acide dans des solvants de faible polarité. Par exemple, la solubilité de l'AlCl₃ en absence d'agent complexant ne dépasse pas 1,5.10⁻³M dans le dichlorométhane. De plus, le système d'amorçage selon l'invention présente une activité accrue d'où la possibilité d'utiliser le système d'amorçage en moindre quantité. On observe donc une augmentation de la cinétique de la réaction avec la libération du centre actif (oxo-carbénium) de son contre-anion et la présence d'un système d'amorçage homogène. On observe également une diminution des réactions de transfert grâce à la capture dudit contre-anion. On obtient ainsi des chaînes à masses molaires plus élevées et une augmentation du rendement.

Un autre avantage des étapes de lavage et d'absorption est d'éviter la présence de traces protiques (AIB) qui pourraient initier des réactions parasites (terminaisons, amorçage protonique...).

Concernant l'amorceur (I), il est choisi dans les amorceurs classiques rentrant dans la composition des systèmes de Friedel et Craft pour la polymérisation cationique des oléfines, il peut être :
- (I1) mono-fonctionnel, c'est à dire présenter une seule fonction chimique et avoir une formule chimique générale suivante :
   R₁-CO-X, R₁-COO-R₂ et R₁-O-R₂ avec les groupements R₁ et R₂ pris dans le groupe comprenant les éléments suivants : un atome d'hydrogène, un groupement alkyl/aryle tel que CH₃, CH₃CH₂, (CH₃)₂CH, (CH₃)₃C, C₆H₅ et des cycles aromatiques substitués, les groupements R₁ et R₂ pouvant être identiques ou différents, et X égale à un atome d'halogène (F, Cl, Br, I) ;
- (I2) di-fonctionnel, c'est à dire présenter deux fonctions chimiques et avoir une formule chimique générale suivante: X₁-CO-R-CO-X₂, R₁-Q-CQ-R-CQ-Q-R₂ avec le groupement R pris dans le groupe comprenant les éléments suivants : un groupement alkyl /aryl tel que CH₃, CH₃CH₂, (CH₃)₂CH, (CH₃)₃C, C₆H₅ et des cycles aromatiques substitués, et les groupements R₁ et R₂ pris dans le groupe comprenant les éléments suivants : un atome d'hydrogène, un groupement alkyl, aryl tel que CH₃, CH₃CH₂, (CH₃)₂CH, (CH₃)₃C, C₆H₅, et des cycles aromatiques substitués, les groupements R₁ et R₂ pouvant être identiques ou différents, et X₁ et X₂ pris dans le groupe contenant F, Cl, Br et I, les groupements X₁ et X₂ pouvant être identiques ou différents ;
- (13) un dérivé halogéné de formule chimique générale suivante : 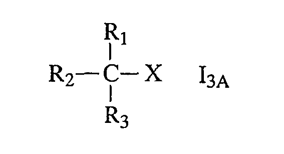 dans laquelle X est un halogène (F, Cl Br ou I), R₁ est sélectionné dans le groupe contenant des groupements alkyl de 1 à 8 atomes de carbone et des groupements alcène ayant de 2 à 8 atomes de carbone, R₂ est sélectionné dans le groupe contenant des groupements alkyl ayant de 4 à 200 atomes de carbone, des groupements alcène, phényl, phénylalkyl (radical en position alkyl), alkylphényl (radical en position phényl) ayant de 2 à 8 atomes de carbone, des groupements cycloalkyl ayant de 3 à 10 atomes de carbone, R₃ est pris dans le groupe comprenant des groupements alkyl ayant de 1 à 8 atomes de carbone et des groupements alcène et phénylalkyl (radical alkyl) ayant de 2 à 8 atomes de carbone, R1, R2, R3 peuvent aussi être de la forme adamantyl ou bornyl avec X étant dans une position de carbone tertiaire ;
   ou de formule chimique générale suivante : 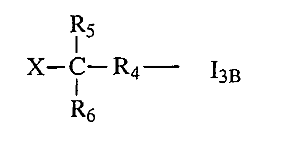 dans laquelle X est halogène (F, Cl, Br ou I), R₅ est pris dans le groupe comprenant des groupements alkyl ayant de 1 à 8 atomes de carbone et des groupements alcène ayant de 2 à 8 atomes de carbone, R6 est pris dans le groupe comprenant des groupements alkyl ayant de 1 à 8 atomes de carbone et des groupements alcène ou phénylakyl (radical alkyl) ayant de 2 à 8 atomes de carbone et R4 pris dans le groupe comprenant les groupements phénylène, biphényl, α,ω-diphénylalcane et -(CH₂)ₙ- avec n un nombre entier de 1 à 10 ;
   ou de formule chimique générale suivante : 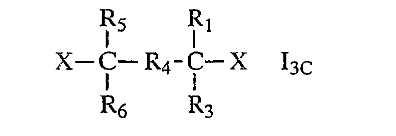 avec X, R₁, R₃, R₄, R₅ et R₆ tels que définis plus haut;
- (I4) un acide protique ou acide de Brönsted comme par exemple CF₃SO₃H, H₂SO₄ ou HClO₄, HBr, HCl, et HI.

On peut citer à titre d'exemples comme amorceurs (I), les esters de cumyl d'acides hydrocarbonés tel que le 2-acétyl-2-phénylpropane, les alkylcumyl éthers tel que 2-méthoxy-2-phénylpropane, 1,4-di(2-méthoxy-2-propyl)benzène, les halogènures de cumyl particulièrement les chlorés tel que le 2-chloro-2-phénylpropane, le (1-chloro-1-méthyléthyl)benzène, le 1,4-di(2-chloro-2-propyl)benzène, le 1,3,5-tri(2-chloro-2-propyl)benzène, les halogénures aliphatiques particulièrement les chlorés tel que le 2-chloro-2,4,4-triméthylpentane (TMPCI), le 2-bromo-2,4,4-triméthylpentane (TMPBr), , le 2,6-dichloro-2,4,4,6-tetraméthylheptane, les hydroxyaliphatiques ou les hydroxycumyls tel que 1,4-di((2-hydroxyl-2-propyl)-benzène), le 2,6-dihydroxyl-2,4,4,6-tetraméthyl-heptane, le 1-chloroadamantane, le 1-chlorobornane, le 5-tert-butyl-1,3-di(1-chloro-1-méthyéthyl)benzène et autres composés similaires.

Concernant le co-amorceur (K), c'est un acide de Lewis, préférentiellement un acide de Lewis fort (tel que par exemple : AlCl₃, AlBr₃, EtAlCl₂, BF₃, BCl₃, SbF₅, SiCl₄) afin de privilégier la structure cétonique, de formule chimique générale RₙMX₃₋ₙ, MX₄ ou MX_{y} selon la nature de l'élément M avec :
- M un élément appartenant aux colonnes IB, IIB et A, IIIB et IIIA, IVB et IVA, VB et VA, VIIIB du tableau de classification périodique des éléments, à titre d'exemples, on peut citer pour M les éléments suivants : B, Ti, Sn, Al, Hf, Zn, Be, Sb, Ga, In, Zr, V, As, Bi. De préférence, M appartient aux colonnes :
   - IIIA (formule RₙMX₃₋ₙ) ;
   - VA et VB (formule MX_{y}) ;
   - IVA et IVB (formule MX₄) ;
- R un radical monovalent pris dans le groupe contenant les groupements hydrocarbonés de 1 à 12 atomes de carbone de type alkyl, aryl, arylalkyl, alkylaryl, cycloalkyl et des alkoxy tels que par exemple les groupements suivants CH₃, CH₃CH₂, (CH₃)₂CH, (CH₃)₃C, C₆H₅, des cycles aromatiques substitués, OCH₃, OC₂H₅, OC₃H₇. Les termes « arylalkyl » et « alkylaryl » font référence à un radical contenant des structures aliphatique et aromatique couplées, le radical étant en position akyl dans le premier cas et aryl dans le second cas.
- X un halogène pris dans le groupe F, Cl, Br et I, de préférence Cl.
- n un nombre entier de 0 à 3 et y un nombre entier de 3 à 5.

A titre d'exemple, on peut citer TiCl₄, ZrCl₄, SnCl₄, VCl₄, SbF₅, AlCl₃, AlBr₃, BF₃, BCl₃, FeCl₃, EtAlCl₂ (abrégé EADC), Et_{1.5}AlCl_{1.5} (abrégé EASC) et Et₂AlCl (abrégé DEAC), AlMe₃ et AlEt₃. Les acides de Lewis peuvent également être supportés sur des argiles, des zéolithes, de la silice ou de la silice alumina, ceci permettant la récupération du système d'amorçage supporté à la fin de la réaction et donc son recyclage.

Les acides de Lewis particulièrement préférés pour notre système de polymérisation cationique sont AlCl₃, AlBr₃, EADC, EASC, DEAC, BF₃, TiCl₄.

Concernant l'agent complexant (CoK), c'est un agent libérant le centre actif de polymérisation du contre-anion généré par la réaction entre le co-amorceur (K) et l'amorceur (I). Le centre actif de polymérisation est ainsi rendu plus accessible grâce à l'action du CoK. L'agent complexant est en particulier un agent complexant servant à complexer le contre-anion généré par la réaction entre le co-amorceur et l'amorceur ce qui a pour effet de libérer le centre actif de polymérisation. On peut citer à titre d'exemples l'o-chloranyl (3,4,5,6-tétrachloro-1,2-benzoquinone), le p-chloranyl (2,3,5,6-tétrachloro-1,4-benzoquinone), le nitrobenzène, le trinitrobenzène, le difluoronitrobenzène, le tétracyanoéthylène, le pentafluorobenzène, l'hexafluorobenzène ou l'octafluorotoluène.

On ne sortirait pas de l'invention si l'on utilisait un agent de transfert et/ou un limiteur de longueur de chaînes bien connu de l'homme de l'art dans le domaine de polymérisation cationique en plus des protagonistes du système d'amorçage cités plus haut.

Nous allons maintenant donner des exemples de préparations du DMK selon l'invention.
La pyrolyse de l'ANIB se fait par simple activation thermique de l'anhydride dans un tube vide selon l'équation suivante : 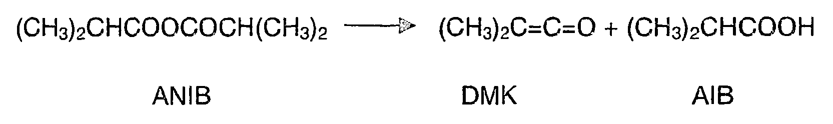 Dans les essais labo, l'ANIB est introduit dans le réacteur dilué dans un gaz inerte (He). Les essais se font à pression atmosphérique. Le réacteur est constitué de deux zones de chauffe (préchauffage du réactif à entre 300 et 340°C et zone de craquage entre 400 et 500°C). Les résultats obtenus au laboratoire sont consignés dans le **tableau 1** ci-dessous.

| | | | | | | |
|---|---|---|---|---|---|---|
| Four zone 1 | 300°C | 300°C | 300°C | 340°C | 340°C | 340°C |
| Four zone 2 | 416°C | 422°C | 404°C | 438°C | 432°C | 484°C |
| Tps cont | 0.22s | 0.22s | 0.21 s | 0.21 s | 0.19s | 0.19s |
| [AnIB] | 8% | 8% | 14% | 14% | 21% | 15% |
| Conversion | 81% | 83% | 50% | 84% | 83% | 91% |
| Sél craquage | 100% | 100% | 100% | 100% | 100% | 100% |
| Bilan C | 103% | 101% | 101% | 102% | 99% | 102% |

| | | | | | | |
|---|---|---|---|---|---|---|
| • Four zone 1 : température en °C de la zone de préchauffage de l'installation. • Four zone 2 : température en °C de la zone de craquage de l'installation. • Tps cont : temps de contact en seconde de la phase gazeuse dans la zone réactionnelle. • [AnIB] : concentration d'ANIB introduit, dilué dans le gaz inerte (He) en % en moles. • Conversion : Taux de conversion = (quantité en moles d'ANIB transformée) / (quantité d'ANIB en moles entrant dans le réacteur) x 100. • Sél craquage: sélectivité de craquage = (nombre de moles de DMK obtenu) / (nombre de moles d'ANIB transformé) x 100. • Bilan C : taux de craquage = (nombre de moles de DMK , d'AIB et d'ANIB sortant du réacteur) / (nombre de moles d'ANIB entrant dans le réacteur) x 100. | | | | | | |

## Revendications

1. Procédé de préparation de diméthylcétène (DMK) par pyrolyse de l'anhydride isobutyrique (ANIB) dans lequel :
a) on préchauffe à pression atmosphérique entre 300 et 340°C un mélange comprenant 1 à 50% en volume d'ANIB pour respectivement 99 à 50% de gaz inerte,
b) puis ce mélange est porté à une température comprise entre 400 et 550°C pendant un temps de contact compris entre 0,05 et 10 s pour obtenir un mélange de DMK, de gaz inerte, d'acide isobutyrique (AIB) voire d'ANIB n'ayant pas réagi,
c) le mélange issu de b) est refroidi (étape de condensation) pour séparer le mélange gazeux comprenant le DMK de l'AIB et/ou de l'ANIB condensés.

2. Procédé selon la revendication 1 dans lequel le gaz inerte est choisi parmi l'azote et l'hélium.

3. Procédé selon l'une quelconque des revendications précédentes dans lequel les proportions d'ANIB et de gaz inerte sont de 5 à 50% en volume d'ANIB pour respectivement 95 à 50% de gaz inerte.

4. Procédé selon l'une quelconque des revendications précédentes dans lequel les proportions d'ANIB et de gaz inerte sont de 8 à 21% en volume d'ANIB pour respectivement 92 à 79% de gaz inerte.

5. Procédé selon l'une quelconque des revendications précédentes dans lequel le temps de contact il est compris entre 0,15 et 0,25 s.

6. Procédé selon l'une quelconque des revendications précédentes dont le mélange gazeux comprenant le DMK et issu de l'étape c) est mis en contact avec une solution de lavage pour réduire voire éliminer les traces d'AIB et/ou d'ANIB comprises dans ledit mélange gazeux et obtenir le DMK avec une pureté suffisante pour le polymériser.

7. Procédé selon la revendication 6, **caractérisé en ce que** le lavage du mélange gazeux comprenant le DMK et issu de l'étape c) s'effectue dans une colonne de lavage munie d'un dévésiculeur.

8. Procédé selon la revendication 7, **caractérisé en ce que** le dévésiculeur est situé soit en tête de colonne soit en pied de colonne, de préférence en tête de colonne.

9. Procédé selon l'une des revendications 7 ou 8, **caractérisé en ce que** le mélange gazeux entre en pied de colonne de lavage, alors que le solvant de lavage entre en tête de colonne.

10. Procédé selon l'une de revendications 6 à 9 dans lequel la solution de lavage est l'ANIB, un hydrocarbure saturé ou insaturé, aliphatique ou alicyclique et substitué ou non substitué.

11. Procédé selon l'une des revendications 1 à 10 dans lequel :
d) le mélange gazeux comprenant le DMK est absorbé dans un solvant de type hydrocarbure saturé ou insaturé, aliphatique ou alicyclique et substitué ou non substitué,
e) puis dans ce solvant contenant le DMK, on effectue la polymérisation en PDMK en présence d'un système d'amorçage cationique comprenant un amorceur (I), un co-amorceur (K) et un agent complexant (CoK),
f) à la fin de la réaction, on élimine le DMK qui n'a pas réagi et on sépare le PDMK du solvant et des restes du système d'amorçage.

12. Procédé selon la revendication 11 dans lequel l'agent complexant (CoK) est un agent libérant le centre actif de polymérisation de son contre-anion généré par la réaction entre le co-amorceur (K) et l'amorceur (I).

13. Procédé selon la revendication 12, **caractérisé en ce que** l'agent complexant (CoK) est une molécule possédant au moins une double liaison appauvrie en électron par un groupement électro-attracteur.

14. Procédé selon la revendication 13 dans lequel l'agent complexant (CoK) est pris dans le groupe de molécules suivantes : l'o-chloranyl (3,4,5,6-tétrachloro-1,2-benzoquinone), le p-chloranyl (2,3,5,6-tétrachloro-1,4-benzoquinone), le nitrobenzène, le trinitrobenzène, le difluoronitrobenzène, le tétracyanoéthylène, le pentafluorobenzène, l'hexafluorobenzène et l'octafluorotoluène.

15. Procédé selon l'une quelconque des revendications 11 à 14 dans lequel le co-amorceur(K) comprend un élément (M) appartenant aux colonnes IB, IIB et A, IIIB et IIIA, IVB et IVA, VB et VA, VIIIB du tableau de classification périodique des éléments.

16. Procédé selon la revendication 15, **caractérisé en ce que** l'élément (M) est pris dans le groupe contenant les éléments chimiques B, Ti, Sn, Al, Hf, Zn, Be, Sb, Ga, In, Zr, V, As et Bi.

17. Procédé selon la revendication 15 ou 16 dans lequel le co-amorceur (K) est un acide de Lewis de formule générale RₙMX₃₋ₙ pour M un élément appartenant à la colonne IIIA, de formule générale MX₄ pour M un élément appartenant aux colonnes VA, IVA et IVB et de formule générale MX₅ pour M un élément appartenant à la colonne VB avec :
- R un radical monovalent pris dans le groupe contenant les groupements hydrocarbonés de 1 à 12 atomes de carbone de type alkyl, aryl, arylalkyl, alkylaryl, cycloalkyl et des alkoxy ;
- X un atome d'halogène pris dans le groupe F, Cl, Br et I ;
- n un nombre entier de 0 à 3.

18. Procédé selon l'une quelconque des revendications 15 à 17 dans lequel le co-amorceur (K) est pris dans le groupe comprenant TiCl₄, ZrCl₄, SnCl₄, VCl₄, SbF₅, AlCl₃, AlBr₃, BF₃, BCl₃, FeCl₃, EtAlCl₂, Et_{1.5}AlCl_{1.5}, Et₂AlCl, AlMe₃ et AlEt₃.

19. Procédé selon l'une quelconque des revendications 11 à 18 dans lequel l'amorceur (I) peut être une molécule monofonctionnelle (11), une molécule difonctionnelle (12), une molécule substituée par un ou des atomes d'halogène (I3) ou un acide de Brönsted (I4).

## Claims

1. Process for the preparation of dimethylketene (DMK) by pyrolysis of isobutyric anhydride (IBAN), in which:
a) a mixture comprising 1 to 50% by volume of IBAN for respectively 99 to 50% of inert gas is preheated at atmospheric pressure between 300 and 340°C,
b) this mixture is then brought to a temperature of between 400 and 550°C for a contact time of between 0.05 and 10 s, in order to obtain a mixture of DMK, of inert gas, of isobutyric acid (IBA) and indeed even of unreacted IBAN,
c) the mixture resulting from b) is cooled (condensation stage), in order to separate the gas mixture comprising the DMK from the condensed IBA and/or condensed IBAN.

2. Process according to Claim 1, in which the inert gas is chosen from nitrogen and helium.

3. Process according to either one of the preceding claims, in which the proportions of IBAN and of inert gas are from 5 to 50% by volume of IBAN for respectively 95 to 50% of inert gas.

4. Process according to any one of the preceding claims, in which the proportions of IBAN and of inert gas are from 8 to 21% by volume of IBAN for respectively 92 to 79% of inert gas.

5. Process according to any one of the preceding claims, in which the contact time is between 0.15 and 0.25 s.

6. Process according to any one of the preceding claims, the gas mixture of which comprising DMK and resulting from stage c) is brought into contact with a washing solution in order to reduce, indeed even remove, the traces of IBA and/or of IBAN included in said gas mixture and to obtain the DMK with a purity sufficient to polymerize it.

7. Process according to Claim 6, **characterized in that** the gas mixture comprising the DMK resulting from stage c) is washed in a washing column equipped with a demister.

8. Process according to Claim 7, **characterized in that** the demister is situated either at the column top or at the column bottom, preferably at the column top.

9. Process according to either of Claims 7 and 8, **characterized in that** the gas mixture enters at the bottom of the washing column, whereas the washing solvent enters at the column top.

10. Process according to one of Claims 6 to 9, in which the washing solution is IBAN or a saturated or unsaturated, aliphatic or alicyclic and substituted or unsubstituted hydrocarbon.

11. Process according to one of Claims 1 to 10, in which:
d) the gas mixture comprising the DMK is absorbed in a solvent of saturated or unsaturated, aliphatic or alicyclic and substituted or unsubstituted hydrocarbon type,
e) the polymerization is then carried out, in this solvent comprising the DMK, to give PDMK in the presence of a cationic initiating system comprising an initiator (I), a coinitiator (K) and a complexing agent (CoK),
f) at the end of the reaction, the unreacted DMK is removed and the PDMK is separated from the solvent and from the residues of the initiating system.

12. Process according to Claim 11, in which the complexing agent (CoK) is an agent which releases the polymerization active center from its counteranion generated by the reaction between the coinitiator (K) and the initiator (I).

13. Process according to Claim 12, **characterized in that** the complexing agent (CoK) is a molecule having at least one double bond depleted in electrons by an electron-withdrawing group.

14. Process according to Claim 13, in which the complexing agent (CoK) is taken from the group of following molecules: o-chloranil (3,4,5,6,-tetrachloro-1,2-benzoquinone), p-chloranil (2,3,5,6-tetrachloro-1,4-benzoquinone), nitrobenzene, trinitrobenzene, difluoronitrobenzene, tetracyanoethylene, pentafluorobenzene, hexafluorobenzene and octafluorotoluene.

15. Process according to any one of Claims 11 to 14, in which the coinitiator (K) comprises an element (M) belonging to Groups IB, IIB and A, IIIB and IIIA, IVB and IVA, VB and VA, and VIIIB of the Periodic Table of the Elements.

16. Process according to Claim 15, **characterized in that** the element (M) is taken from the group consisting of the chemical elements B, Ti, Sn, Al, Hf, Zn, Be, Sb, Ga, In, Zr, V, As and Bi.

17. Process according to Claim 15 or 16, in which the coinitiator (K) is a Lewis acid of general formula RₙMX₃₋ₙ for M an element belonging to Group IIIA, of general formula MX₄ for M an element belonging to Groups VA, IVA and IVB and of general formula MX₅ for M an element belonging to Group VB, with:
- R a monovalent radical taken from the group consisting of the hydrocarbon groups of 1 to 12 carbon atoms of alkyl, aryl, arylalkyl, alkylaryl or cycloalkyl type and alkoxys;
- X a halogen atom taken from the group consisting of F, Cl, Br and I;
- n an integer from 0 to 3.

18. Process according to any one of Claims 15 to 17, in which the coinitiator (K) is taken from the group consisting of TiCl₄, ZrCl₄, SnCl₄, VCl₄, SbF₅, AlCl₃, AlBr₃, BF₃, BCl₃, FeCl₃, EtAlCl₂, Et_{1.5}AlCl_{1.5}, Et₂AlCl, AlMe₃ and AlEt₃.

19. Process according to any one of Claims 11 to 18, in which the initiator (I) can be a monofunctional molecule (I1), a difunctional molecule (12), a molecule substituted by one or more halogen atoms (13) or a Brönsted acid (I4).

## Patentansprüche

1. Verfahren zur Herstellung von Dimethylketen (DMK) durch Pyrolyse von Isobuttersäureanhydrid (IBAN), bei dem man:
a) ein 1 bis 50 Vol.-% IBAN auf 99 bis 50% Inertgas enthaltendes Gemisch bei Normaldruck auf eine Temperatur zwischen 300 und 340°C erhitzt,
b) dieses Gemisch dann für eine Kontaktzeit zwischen 0,05 und 10 s auf eine Temperatur zwischen 400 und 550°C bringt, wobei man ein Gemisch aus DMK, Inertgas, Isobuttersäure (IBA) und nicht umgesetztem IBAN erhält,
c) das Gemisch aus b) abkühlt (Kondensationsschritt), um das das DMK enthaltende Gasgemisch von der kondensierten IBA und/oder dem kondensierten IBAN zu trennen.

2. Verfahren nach Anspruch 1, bei dem man das Inertgas unter Stickstoff und Helium auswählt.

3. Verfahren nach einem der vorhergehenden Ansprüche, bei dem sich die Anteile an IBAN und Inertgas auf 5 bis 50 Vol.-% IBAN auf 95 bis 50% Inertgas belaufen.

4. Verfahren nach einem der vorhergehenden Ansprüche, bei dem sich die Anteile an IBAN und Inertgas auf 8 bis 21 Vol.-% IBAN auf 92 bis 79% Inertgas belaufen.

5. Verfahren nach einem der vorhergehenden Ansprüche, bei dem die Kontaktzeit zwischen 0,15 und 0,25 s liegt.

6. Verfahren nach einem der vorhergehenden Ansprüche, bei dem man das das DMK enthaltende Gasgemisch aus Schritt c) mit einer Waschlösung in Kontakt bringt, um darin enthaltene Spuren von IBA und/oder IBAN teilweise oder ganz zu eliminieren und das DMK mit einer für die Polymerisation ausreichenden Reinheit zu erhalten.

7. Verfahren nach Anspruch 6, **dadurch gekennzeichnet, daß** das Waschen des das DMK enthaltenden Gasgemischs aus Schritt c) in einer Waschkolonne mit Demister erfolgt.

8. Verfahren nach Anspruch 7, **dadurch gekennzeichnet, daß** sich der Demister entweder am Kolonnenkopf oder am Kolonnenboden befindet, vorzugsweise am Kolonnenkopf.

9. Verfahren nach Anspruch 7 oder 8, **dadurch gekennzeichnet, daß** das Gasgemisch am Boden der Waschkolonne, das Waschlösungsmittel hingegen am Kolonnenkopf eintritt.

10. Verfahren nach einem der Ansprüche 6 bis 9, bei dem es sich bei der Waschlösung um IBAN oder einen gesättigten oder ungesättigten, aliphatischen oder alicyclischen und substituierten oder unsubstituierten Kohlenwasserstoff handelt.

11. Verfahren nach einem der Ansprüche 1 bis 10, bei dem man:
d) das das DMK enthaltende Gasgemisch in einem Lösungsmittel vom Typ gesättigter oder ungesättigter, aliphatischer oder alicyclischer und substituierter oder unsubstituierter Kohlenwasserstoff absorbiert,
e) dann in diesem das DMK enthaltenden Lösungsmittel die Polymerisation zu PDMK in Gegenwart eines kationischen Initiatorsystems mit einem Initiator (I), einem Coinitiator (K) und einem Komplexbildner (CoK) durchführt,
f) am Ende der Reaktion das nicht umgesetzte DMK eliminiert und das PDMK von dem Lösungsmittel und den Resten des Initiatorsystems abtrennt.

12. Verfahren nach Anspruch 11, bei dem es sich bei dem Komplexbildner (CoK) um ein Mittel handelt, welches das aktive Polymerisationszentrum aus dessen durch Reaktion zwischen dem Coinitiator (K) und dem Initiator (I) gebildeten Gegenanion freisetzt.

13. Verfahren nach Anspruch 12, **dadurch gekennzeichnet, daß** es sich bei dem Komplexbildner (CoK) um ein Molekül mit mindestens einer durch eine elektronenanziehende Gruppe elektronenarm gemachten Doppelbindung handelt.

14. Verfahren nach Anspruch 13, bei dem der Komplexbildner (CoK) aus der Gruppe der folgenden Moleküle stammt: o-Chloranil (3,4,5,6,-Tetrachlor-1,2-benzochinon), p-Chloranil (2,3,5,6-Tetrachlor-1,4-benzochinon), Nitrobenzol, Trinitrobenzol, Difluornitrobenzol, Tetracyanoethylen, Pentafluorbenzol, Hexafluorbenzol und Octafluortoluol.

15. Verfahren nach einem der Ansprüche 11 bis 14, bei dem der Coinitiator (K) ein Element (M) aus den Gruppen IB, IIB und A, IIIB und IIIA, IVB und IVA, VB und VA und VIIIB des Periodensystems der Elemente enthält.

16. Verfahren nach Anspruch 15, **dadurch gekennzeichnet, daß** das Element (M) aus der Gruppe enthaltend die chemischen Elemente B, Ti, Sn, Al, Hf, Zn, Be, Sb, Ga, In, Zr, V, As und Bi stammt.

17. Verfahren nach Anspruch 15 oder 16, bei dem es sich bei dem Coinitiator (K) um eine Lewis-Säure mit der allgemeinen Formel RₙMX₃₋ₙ, wenn M für ein Element aus der Gruppe IIIA steht, der allgemeinen Formel MX₄, wenn M für ein Element aus den Gruppen VA, VIA und IVB steht, und der allgemeinen Formel MX₅, wenn M für ein Element aus der Gruppe VB steht, wobei:
- R für einen einwertigen Rest aus der Gruppe enthaltend Kohlenwasserstoffgruppen mit 1 bis 12 Kohlenstoffatomen vom Typ Alkyl, Aryl, Arylalkyl, Alkylaryl, Cycloalkyl und Alkoxy steht;
- X für ein Halogenatom aus der Gruppe F, Cl, Br und I steht;
- n für eine ganze Zahl von 0 bis 3 steht.

18. Verfahren nach einem der Ansprüche 15 bis 17, bei dem der Coinitiator (K) aus der Gruppe enthaltend TiCl₄, ZrCl₄, SnCl₄, VCl₄, SbF₅, AlCl₃, AlBr₃, BF₃, BCl₃, FeCl₃, EtAlCl₂, Et_{1,5}AlCl_{1,5}, Et₂AlCl, AlMe₃ und AlEt₃ steht.

19. Verfahren nach einem der Ansprüche 11 bis 18, bei dem es sich bei dem Initiator (I) um ein monofunktionelles Molekül (I1), ein difunktionelles Molekül (12), ein durch ein oder mehrere Halogenatome substituiertes Molekül (13) oder eine Brönsted-Säure (14) handeln kann.
